# EUROPEAN PATENT APPLICATION

(11) **EP 4 122 526 A1**
(43) Date of publication of application: **25.01.2023**
(21) Application number: 22186232.9
(22) Date of filing: 21.07.2022
(51) Int. Cl.: A61M 39/24, A61M 39/26

(54) **COUPLER DEVICE FOR VALVE ASSEMBLY FOR USE WITH MEDICAL INFUSION DEVICE**

(30) Priority: 21.07.2021 US 202163224190 P
(71) Applicant: Nexus Medical, LLC, Lenexa, KS 66214 (US)
(72) Inventor: DIKEMAN, W. Cary, Leawood, 66209 (US); WILSON, Taylor, Lee's Summit, 64086 (US)
(74) Representative: Gill Jennings & Every LLP

(57) **Abstract**

A coupler of a valve assembly for coupling a flow control valve to an infusion device. The coupler includes a proximal portion and a distal portion. The proximal portion comprises a plurality of recessed segments configured for receiving the infusion device. The distal portion comprises a valve flange that defines a central cavity for receiving the flow control valve. The valve flange comprises a swage extending radially inward to the central cavity for mechanically engaging a luer lock fitting of the valve assembly.

## Description

### CROSS-REFERENCE TO RELATED APPLICATIONS

This application claims the benefit of U.S. Provisional Application No. 63/224,190 filed on July 21, 2021, entitled "Coupler Device for Valve Assembly for Use with Medical Infusion Device", the entire contents of which are herein incorporated by reference.

### BACKGROUND

### 1. FIELD

Embodiments of the invention relate generally to pressure-activated infusion devices used for the administration of fluids to patients. More specifically, embodiments of the present invention are directed to a coupler component designed to connect a flow-control valve to an infusion device.

### 2. RELATED ART

The use of infusion devices for the administration of parenteral and other fluids to patients is a common practice. A variety of devices for such purposes have been proposed in the past, such as a simple length of tubing having a fitting on one end for making connection with a source of fluid (e.g., a bottle or flexible bag), while the other end is provided with a needle or catheter which may be inserted into the vein of a patient. More commonly, however, specialized infusion devices are provided which include a venous needle (or catheter) at one end and a septum at the other end. In the use of these devices, the needle (or catheter) is inserted into the patient and the device is taped or otherwise affixed to the patient or adjacent equipment. Thereupon, a cannula connected to a liquid supply may be inserted into the free septum end of the device in order to begin fluid therapy. The septum provides a swabable injection site that can be reused, while the needle (or catheter) remains inserted into the patient. A persistent problem with prior infusion devices is referred to as fluid reflux, or the tendency for fluids, such as blood or medication, to be drawn into the infusion apparatus. Fluid reflux can occur in prior art devices, for example, when a gravity supply fluid source is empty, when medication is infused through an adjacent component, or when a cannula is removed from a septum or port. In an attempt to prevent fluid reflux, pressure-activated infusion devices are used.

Prior art pressure-activated infusion devices that reduce blood reflux using a flexible check valve are problematic due to precise coupling of the valve to the infusion device. Coupling of flexible check valves to pressure-activated infusion devices is notoriously difficult due to required alignment of the internal passage with the valve housing. Prior art check valves and associated couplers are also known to shift or "squirm" within the housing, often when the valve is seated in the housing. This inadvertent movement can cause valve misalignment and improper operation.

There is accordingly a need in the art for improved pressure-activated infusion devices equipped with a specific coupler to properly align and secure the valve component to the pressure-activated infusion device, which eliminates the possibility of blood reflux and can be reliably manufactured.

### SUMMARY

This summary is provided to introduce a selection of concepts in a simplified form that are further described below in the detailed description. This summary is not intended to identify key features or essential features of the claimed subject matter, nor is it intended to be used to limit the scope of the claimed subject matter. Other aspects and advantages of the invention will be apparent from the following detailed description of the embodiments and the accompanying drawing figures.

### BRIEF DESCRIPTION OF THE DRAWING FIGURES

Embodiments of the invention are described in detail below with reference to the attached drawing figures, wherein:
FIG. 1 is a perspective view of a valve assembly constructed in accordance with a first embodiment of the present invention.
FIG. 2 is an exploded proximal perspective view of the first embodiment of the valve assembly, particularly showing a coupler, a flow control valve, and a luer lock fitting.
FIG. 3 is a cross-sectional view of the first embodiment of the valve assembly.
FIG. 4 is a magnified cross-sectional view of the first embodiment of the valve assembly.
FIG. 5 is a cross-sectional view of the first embodiment of the coupler shown in FIG. 1.
FIG. 6 is a distal perspective of the first embodiment of the coupler shown in FIG. 1.
FIG. 7 is a proximal perspective view of the first embodiment of the coupler shown in FIG. 1.
FIG. 8 is a cross-sectional view of the first embodiment of the valve assembly shown in FIG. 1 coupled to an infusion device.
FIG. 9 is an exploded distal perspective view of the first embodiment of the valve assembly and the infusion device of FIG. 8.

The drawing figures do not limit the invention to the specific embodiments disclosed and described herein. The drawings are not necessarily to scale, emphasis instead being placed upon clearly illustrating the principles of the invention.

### DETAILED DESCRIPTION

The following detailed description references the accompanying drawings that illustrate specific embodiments in which the invention can be practiced. The embodiments are intended to describe aspects of the invention in sufficient detail to enable those skilled in the art to practice the invention. Other embodiments can be utilized and changes can be made without departing from the scope of the invention. The following detailed description is, therefore, not to be taken in a limiting sense. The scope of the invention is defined only by the appended claims, along with the full scope of the equivalents to which such claims are entitled.

In this description, references to "one embodiment," "an embodiment," or "embodiments" mean that the feature or features being referred to are included in at least one embodiment of the technology. Separate references to "one embodiment," "an embodiment," or "embodiments" in this description do not necessarily refer to the same embodiment and are also not mutually exclusive unless stated and/or except as will be readily apparent to those skilled in the art from the description. For example, a feature, structure, act, etc. described in one embodiment may also be included in other embodiments, but is not necessarily included. Thus, the technology can include a variety of combinations and/or integrations of the embodiments described herein.

Embodiments of the invention are directed to a coupler for a valve assembly that couples an infusion device to a flow control valve. Particularly, the coupler may operatively connect a pressure-activated infusion device to a flexible pressure-actuated flow control valve whereby the connection may regulate infusion of medical liquids into a patient or aspiration of blood from the patient.

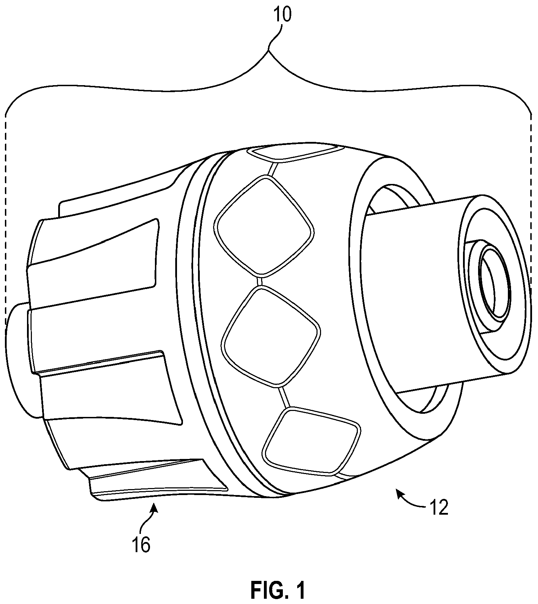

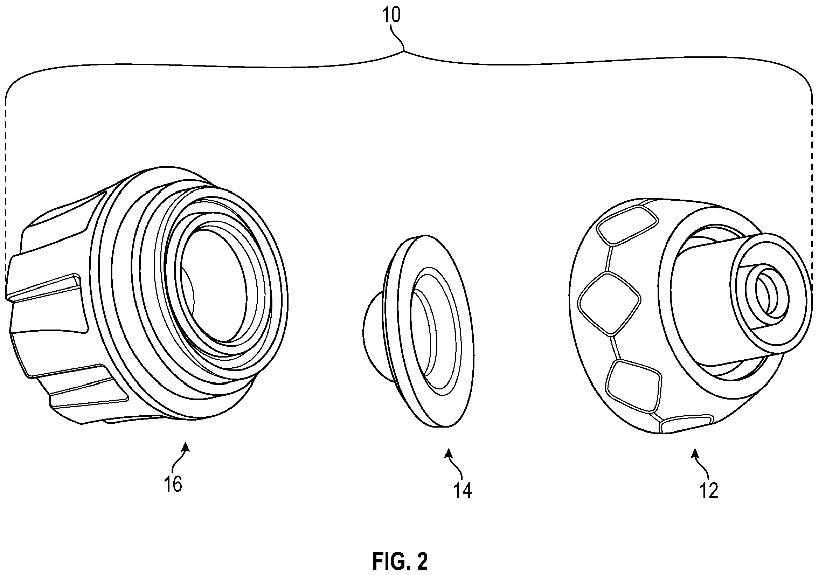

Figure 1 shows a first embodiment of valve assembly 10. The valve assembly 10 may include a coupler 12 and a luer lock fitting 16. As can be seen in Figure 2, the valve assembly 10 may also include a flow control valve 14 housed in between the coupler 12 and luer lock fitting 16. In some embodiments, flow control valve 14 may be pressure activated, such that it opens and closes based on a particular pressure differential. In some embodiments, flow control valve 14 may be a diaphragm. In some embodiments, flow control valve 14 may be comprised of a medical grade elastomeric material, such as a silicone elastomer.

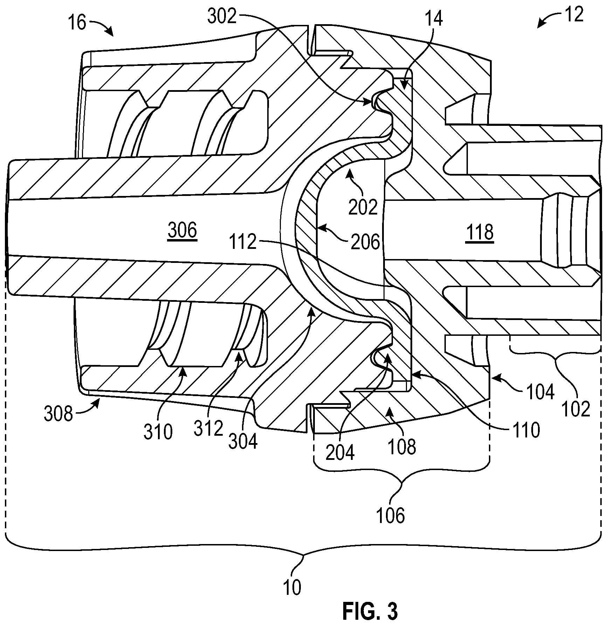

With respect to Figure 3, the coupler 12 includes a proximal circumferential wall 102, a cannula lumen 118, an axial wall 104, and a distal circumferential wall 106. The distal circumferential wall 106 has a valve flange 108 extending distally to receive flow control valve 14 therein. Extending from the valve flange 108, the coupler 12 includes a valve wall 110 and a housing protrusion 112, which operatively engage the flow control valve 14.

In some embodiments, the coupler 12 may comprise an elastomeric medical grade synthetic resin material. In some embodiments, the coupler 12 may comprise a medical grade rigid or semirigid synthetic resinous material suitable for supporting an operable connection, such as, for example, polyvinyl chloride or polycarbonate.

The flow control valve 14 has a valve surface 202 and a slit 206. In some embodiments, the valve surface 202 may have a convex form. In some embodiments, the valve surface 202 may have a concave form. In some embodiments, the flow control valve 14 is preferably designed to selectively prevent fluid flow in the proximal direction. More particularly, the flow control valve 14 prevents proximal flow when an aspiration pressure differential (i.e., where the pressure against the convex surface of the flow control valve 14 is greater than the pressure against the concave surface of the flow control valve 14) across the flow control valve 14 is below a set aspiration amount. The set aspiration amount is generally greater than the venous pressure (relative to atmosphere pressure) of the patient when fluid is not being injected or aspirated through the injection site. Thus, when the flow control valve 14 experiences the typical venous pressure of the patient, the corresponding aspiration pressure differential is less than the set aspiration amount and is not sufficient to open the flow control valve 14. However, when it is desired to aspirate fluid across the flow control valve 14, fluid can be drawn through the injection site by reducing the fluid pressure on a proximal side of the flow control valve 14 (e.g., by drawing fluid with a syringe) so that the aspiration pressure differential exceeds the set aspiration amount. This causes the flow control valve 14 to open and allow aspiration flow through the cannula lumen 118. In some embodiments, the flow control valve 14 is designed to selectively prevent fluid flow in the distal direction. More particularly, the flow control valve 14 prevents distal flow when an infusion pressure differential (i.e., where the pressure against the concave surface of the flow control valve 14 is greater than the pressure against the convex surface of the flow control valve 14) across the flow control valve 14 is below a set infusion amount. When an external pressure is applied to a proximal side of the flow control valve 14 (e.g., by injection fluid from a syringe or other fluid supply) and the infusion pressure differential exceeds the set infusion amount, the flow control valve 14 opens to allow infusion flow through a central fluid passageway 306. In some embodiments, flow control valve 14 may be configured so that the set aspiration pressure differential required to open the flow control valve 14 is greater than the set infusion pressure differential required to open the flow control valve 14. In an embodiment, the valve surface 202 includes a valve protrusion 204 on the periphery of the flow control valve 14. Valve protrusion 204 may operatively engage the luer lock fitting 16 by being received within the flow valve locking groove 302.

The luer lock fitting 16 includes a central fluid passageway 306 and a rotatable threaded collar 308. In one embodiment, the central fluid passageway 306 allows for the flow of fluids in either the distal or the proximal direction when the flow control valve 14 is activated. The rotatable threaded collar 308 includes an outer body 310 having an internal threading 312, which may be coupled with another threaded medical device.

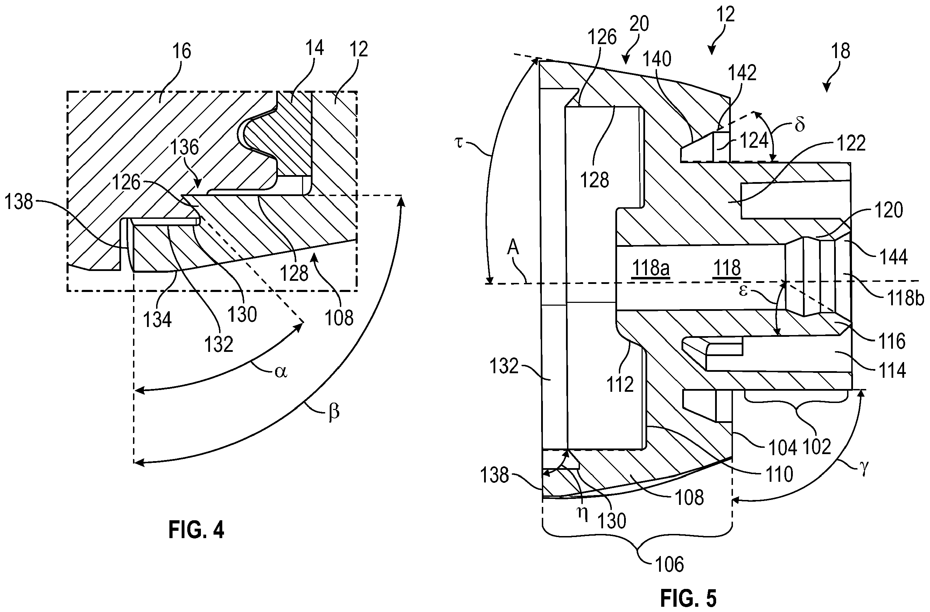

Referring now to Figure 4, the coupler 12, the flow control valve 14, and the luer lock fitting 16 are operatively coupled together. In some embodiments, the coupler 12, flow control valve 14, and luer lock fitting 16 may be welded together to form a permanent connection. The valve flange 108 includes an exterior portion 134, an interior portion 136, and an axial wall 138. Interior portion 136 includes a proximal face 128 and a distal face 132, separated by a swage 126 and a swage recess 130. The swage 126 and swage recess 130 may couple to the luer lock fitting 16. In one embodiment, a welded configuration is achieved by ultrasonically welding the swage 126 to the luer lock fitting 16.

In one embodiment, the swage 126 forms an angle alpha between about 35-55 degrees in from the axial wall 138. In one embodiment, the swage 126 forms an angle alpha of between about 40-50 degrees from the axial wall 138. In one embodiment, the swage 126 forms an angle alpha of about 45 degrees from the axial wall 138.

In one embodiment, the proximal face 128 and the distal face 132 form an angle beta of between about 80-100 degrees with respect to the axial wall 138. In one embodiment, the proximal face 128 and the distal face 132 form an angle beta of between about 85-95 degrees with respect to the axial wall 138. In one embodiment, the proximal face 128 and the distal face 132 form an angle beta of about 89.5 degrees with respect to the axial wall 138.

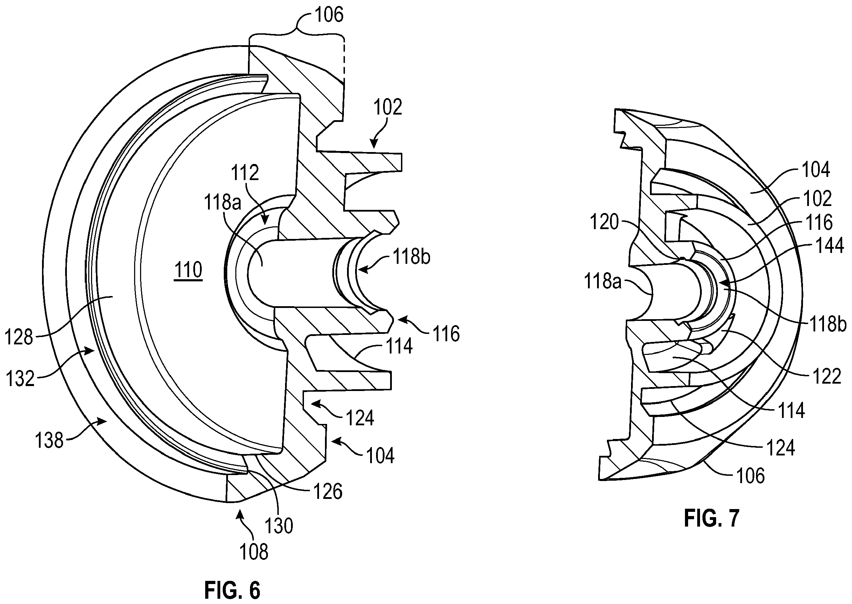

Referring now to Figures 5 and 7, the coupler 12 includes a proximal portion 18 and a distal portion 20. In one embodiment, the proximal portion 18 is designed to couple to an infusion device. In some embodiments, the distal portion 20 is designed to couple to the flow control valve 14 and the luer lock fitting 16, as shown in Figures 3-4. The proximal portion 18 includes the proximal circumferential wall 102 and the axial wall 104, wherein the axial wall 104 forms an angle gamma of between about 80-100 degrees with respect to the proximal circumferential wall 102. In some embodiments, the axial wall 104 forms an angle gamma of about 90 degrees with respect to the proximal circumferential wall 102. In one embodiment, the outer portion of the axial wall 104 is between about 0.3 to 0.5 inches in diameter. In one embodiment, the outer portion of the axial wall 104 is between about 0.35 and 0.45 inches in diameter. In one embodiment, the outer portion of the axial wall 104 is about 0.39 inches.

In one embodiment, the proximal circumferential wall 102 extends proximally to the axial wall 104 a distance between about 0.08 to about 0.16 inches. In one embodiment, the proximal circumferential wall 102 extends proximally to the axial wall 104 a distance between about 0.1 to about 0.14 inches. In one embodiment, the proximal circumferential wall 102 extends proximally to the axial wall 104 a distance of about 0.12 inches.

Between the proximal circumferential wall 102 and the axial wall 104 is a coupling recess 124. In one embodiment, the coupling recess 124 may operatively engage a housing of the infusion device. In one embodiment, the coupling recess 124 contains an angled portion 140 and a flat portion 142. In one embodiment, the angled portion 140 extends at an angle delta of between about 20 to about 30 degrees from the proximal circumferential wall 102. In one embodiment, the angled portion 140 extends at an angle delta of about 25 degrees from the proximal circumferential wall 102.

In one embodiment, the coupling recess 124 extends between about 0.025 to about 0.075 inches distally from the axial wall 104. In one embodiment, the coupling recess 124 extends between about 0.04 to about 0.06 inches distally from the axial wall 104. In one embodiment, the coupling recess 124 extends about 0.05 inches distally from the axial wall 104.

In one embodiment, the diameter of the flat portion 142 of the coupling recess 124 is between about 0.2 to about 0.4 inches. In one embodiment, the diameter of the flat portion 142 of the coupling recess 124 is between about 0.25 to about 0.35 inches. In one embodiment, the diameter of the flat portion 142 of the coupling recess 124 is about 0.31 inches.

The proximal circumferential wall 102 defines a septum 114 located between the proximal circumferential wall 102 and a cannula flange 116. In one embodiment, the proximal circumferential wall 102 is between about 0.02 to about 0.04 inches thick. In one embodiment, the proximal circumferential wall 102 is about 0.03 inches thick.

The septum 114 is designed to operatively couple to a spring from the infusion device. In one embodiment, the width of the septum 114 is between about 0.04 to about 0.12 inches. In one embodiment, the width of the septum 114 is between about 0.06 to about 0.1 inches. In one embodiment, the width of the septum is about 0.08 inches.

On the distal side of the septum 114 are a plurality of septum protrusions 122. In one embodiment, there are between about two to five septum protrusions 122. In one embodiment, there are three septum protrusions 122. In one embodiment, the three septum protrusions 122 are separated by about 120 degrees equally around the septum 114. In one embodiment, the septum protrusions 122 are between about 0.02 to about 0.06 inches wide. In one embodiment, the septum protrusions are about 0.04 inches wide.

The cannula flange 116 defines a cannula lumen 118 which includes a distal cannula lumen 118a and a proximal cannula lumen 118b. In one embodiment, the cannula flange 116 may extend a shorter distance proximally than the proximal circumferential wall 102 from the septum 114. In one embodiment, the cannula flange 116 may extend a longer distance proximally than the proximal circumferential wall 102 from the septum 114. In one embodiment, the cannula flange 116 may extend the same distance proximally from the septum 114 as the proximal circumferential wall 102. In one embodiment, the cannula flange 116 defines a diameter of between about 0.05 to about 0.11 inches. In one embodiment, the cannula flange 116 defines a diameter of between about 0.06 to about 0.1 inches. In one embodiment, the cannula flange 116 defines a diameter of about 0.08 inches.

In one embodiment, the proximal cannula lumen 118b contains an angled wall 144 forming an opening, wherein the angled wall 144 forms an angle epsilon of between about 25-35 degrees with respect to the exterior wall of the cannula flange 116. In one embodiment, the angled wall 144 forms an angle epsilon of about 35 degrees with respect to the exterior wall of the cannula flange 116.

The proximal cannula lumen 118b further includes a transverse groove 120. In one embodiment, the transverse groove 120 is located between about 0.01 to about 0.09 inches distal to the most proximal part of the cannula flange 116. One section of said transverse groove 120 protrudes into the interior wall of the cannula flange 116. In one embodiment, the transverse groove 120 defines a diameter between about 0.05 to about 0.11 inches. In one embodiment, the transverse groove 120 defines a diameter between about 0.08 to about 0.1 inches. In one embodiment, the transverse groove 120 defines a diameter of about 0.09 inches.

In one embodiment, the distal cannula lumen 118a may be angled slightly in the interior direction to define a smaller diameter than the proximal cannula lumen 118b. In one embodiment, the distal end of the distal cannula lumen 118a defines a diameter between about 0.03 to about 0.1 inches. In one embodiment, the distal end of the distal cannula lumen 118a defines a diameter between about 0.05 to about 0.09 inches. In one embodiment, the distal end of the distal cannula lumen 118a defines a diameter of about 0.07 inches.

Referring now to Figures 5 and 6, the coupler 12 includes a distal portion 20 which operatively engages the flow control valve 14 and the luer lock fitting 16. The distal portion 20 includes a distal circumferential wall 106. In one embodiment, the exterior of the distal circumferential wall 106 protrudes at an angle zeta of between about 1 to about 20 degrees from central axis A. In one embodiment, the exterior of the distal circumferential wall 106 protrudes at an angle zeta of between about 5 to about 15 degrees from the central axis A. In one embodiment, the distal circumferential wall 106 protrudes at an angle zeta of about 10 degrees from the central axis A.

In one embodiment, the distal circumferential wall 106 extends between about 0.1 and about 0.3 inches distally from the axial wall 104. In one embodiment, the distal circumferential wall 106 extends between about 0.15 to about 0.25 inches distally from the axial wall 104. In one embodiment, the distal circumferential wall extends about 0.21 inches distally from the axial wall 104.

The distal circumferential wall 106 further includes the valve flange 108 and axial wall 138. The axial wall 138 extends interiorly from the valve flange 108 at an angle eta of about 90 degrees. In one embodiment, the axial wall 138 is between about 0.03 to about 0.07 inches wide. In one embodiment, the axial wall 138 is between about 0.04 to about 0.06 inches wide. In one embodiment, the axial wall 138 is between about 0.053 to about 0.056 inches wide.

The interior portion 136 of the valve flange 108 includes the proximal face 128 and the distal face 132. As discussed above, the proximal face 128 and the distal face 132 are separated by the swage 126 and the swage recess 130. In one embodiment, the distal face 132 defines a diameter between about 0.38 to about 0.44 inches. In one embodiment, the distal face 132 defines a diameter between about 0.4 to about 0.42 inches. In one embodiment, the distal face 132 defines a diameter of about 0.414 inches.

The proximal face 128 defines the outer diameter of the valve wall 110. In one embodiment, the diameter of the proximal face 128 is between about 0.35 and about 0.4 inches. In one embodiment, the diameter of the proximal face 128 is between about 0.36 and about 0.39 inches. In one embodiment, the diameter of the proximal face 128 is about 0.374 inches.

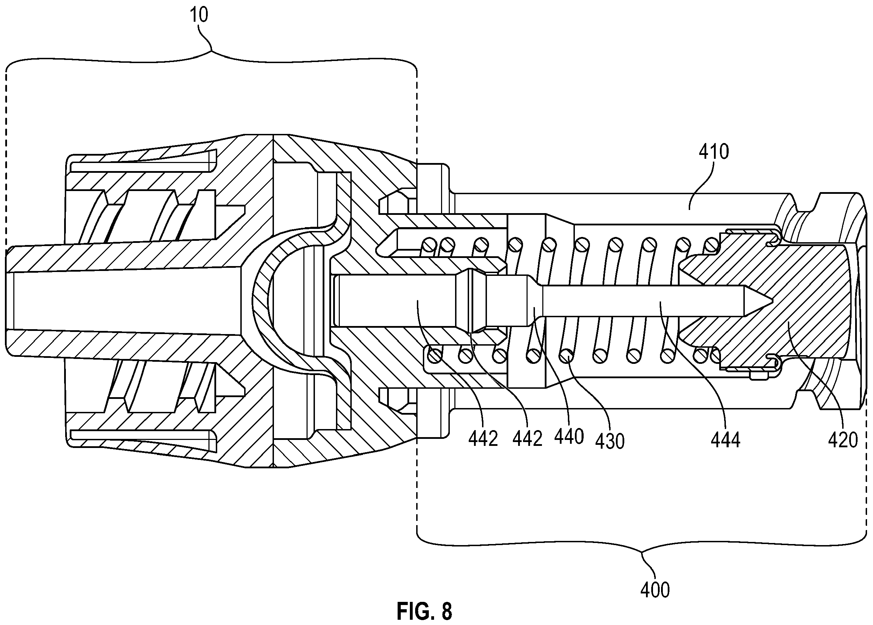

Referring now to Figure 8, the valve assembly 10 is shown coupled to an infusion device 400. Said infusion device 400 may comprise a cannula 440. Cannula 440 may include a distal segment 442 and a proximal segment 444. In some embodiments, the distal segment 442 is received into the cannula lumen 118 of the coupler 12. In some embodiments, the distal segment 442 may comprise a circumferential notch 446 which cooperatively engages the transverse groove 120 of the coupler 12. In some embodiments, the proximal segment 444 extends proximally from the cannula lumen 118 and cooperatively engages a stopper 420.

The stopper 420 may operatively engage a spring 430 of the infusion device 400. The stopper 420 may be designed such that when a force is applied from the proximal end, (i.e., a syringe pushing against the stopper 420) the stopper 420 may compress the spring 430 in the distal direction therein exposing the proximal segment 444 of the cannula 440. Exposure of the cannula 440 may allow for the transfer of fluids through the infusion device 400 and the valve assembly 10 based on pressures exerted on the flow control valve 14.

The spring 430 may operatively engage the coupler 12 by mechanically coupling to the septum 114. The septum 114 is designed to house the spring 430 such that when mechanical force is exerted on the spring 430 through the stopper 420, the spring 430 will not adjust in the lateral direction due to the proximal circumferential wall 102 and the cannula flange 116.

The infusion device 400 may include an infusion device housing 410. The infusion device housing 410 may define a cavity within which the cannula 440, spring 430, and stopper 420 are housed. The infusion device housing 410 may mechanically couple to the coupler 12 through a plurality of surfaces. In one embodiment, the infusion device housing 410 may comprise a protrusion on the proximal side which mechanically engages the coupling recess 124 of the coupler 12. In one embodiment, the infusion device housing 410 may include a flat portion on the proximal side which may mechanically engage the axial wall 104 of the coupler 12.

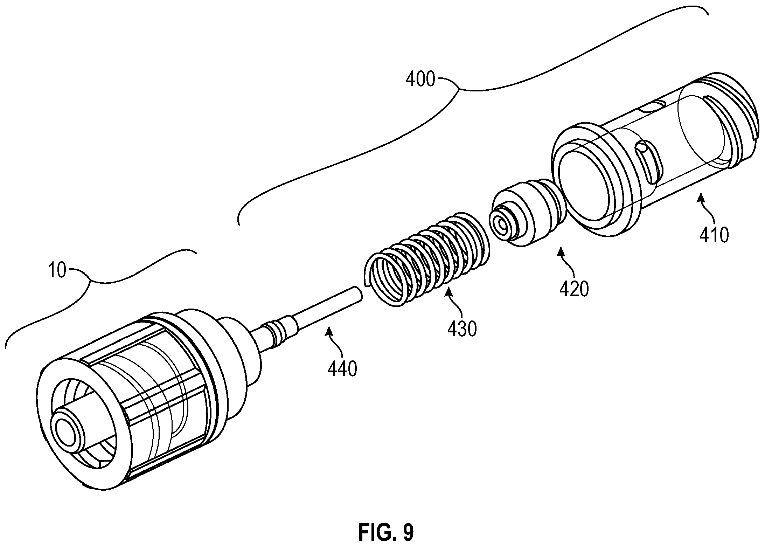

Referring now to Figure 9, the valve assembly 10 and the infusion device 400 are shown in an exploded view. The valve assembly 10 is shown housing the cannula 440 in the cannula lumen 118. The spring 430 may surround the cannula when operatively engaged with the coupler 12 of the valve assembly 10. The stopper 420 may operatively engage the spring 430 and mechanically engage the proximal segment 444 of the cannula 440. The infusion device housing 410, which defines a central cavity, may house the cannula 440, the spring 430, and the stopper 420. The infusion device housing 410 may mechanically couple to the valve assembly 10 via the coupler 12 to keep all parts of the valve assembly 10 and infusion device 400 operatively engaged.

Referring back to Figures 1-4, the valve assembly 10 may be assembled via a plurality of steps to operatively connect the luer lock fitting 16, the flow control valve 14, and the coupler 12. In a first step, the flow control valve 14 is received by the luer lock fitting 16 in the valve recess 304. When correctly received, the valve protrusion 204 will mechanically couple to the flow valve locking groove 302. In a second step, the coupler 12 is placed over the flow control valve 14 and received by the luer lock fitting 16. Specifically, the valve flange 108 may cover, circumferentially, the flow control valve 14. In a third step, the swage 126 is mechanically coupled to the luer lock fitting 16. In one embodiment, this third step comprises welding, such as ultrasonically, the swage 126 to the luer lock fitting 16. In some embodiments, the valve assembly may be constructed using adhesive, mechanical fasteners, welding, or any other mechanical coupling method. It will be appreciated by one skilled in the art that the valve assembly may further include any commonly known method of mechanically coupling devices.

Features described above as well as those claimed below may be combined in various ways without departing from the scope hereof. The following examples illustrate some possible, non-limiting combinations:
(A1) A coupler for a valve assembly configured to couple to an infusion device, the coupler including: a proximal portion, a distal portion, and a cylindrical central passage connecting the proximal portion and the distal portion, the proximal portion including: a plurality of recessed segments configured to receive the infusion device, a first recessed segment of the plurality of recessed segments is configured to engage a spring of the infusion device, a second recessed segment of the plurality of recessed segments is configured to engage a housing of the infusion device, a third recessed segment of the plurality of recessed segments is configured to engage a cannula of the infusion device; and the distal portion including: a central cavity defined by a valve flange, the central cavity is configured to engage a flow control valve.
(A2) For the coupler denoted as (A1), the proximal portion further includes an axial wall configured to engage the housing of the infusion device.
(A3) For the coupler denoted as (A1) or (A2), the valve flange includes a proximal face and a distal face defining a swage therebetween, the swage protruding inwardly into the central cavity.
(A4) For the coupler denoted as any of (A1) through (A3), the swage is configured to engage a luer lock fitting.
(A5) For the coupler denoted as any of (A1) through (A4), the first recessed segment includes a plurality of septum protrusions extending proximally from a distalmost area of the first recessed segment.
(A6) For the coupler denoted as any of (A1) through (A5), the second recessed segment includes an angled portion and a flat portion, the angled portion being radially angled outward at a proximal end of the second recessed segment; and the flat portion extending proximally from the angled portion.
(A7) For the coupler denoted as any of (A1) through (A6), the third recessed segment includes: a transverse groove and an angled wall proximal to the transverse groove, a center of the transverse groove defining a larger diameter than a diameter of the third recessed segment, and the angled wall tapering radially outward, the transverse groove and the angled wall are configured to engage the cannula of the infusion device.
(B1) A system for controlling the flow of liquids to and from a patient, including: a valve assembly and an infusion device, the infusion device including a housing, a cannula, and a spring; the valve assembly including a coupler and a flow control valve; the coupler including a proximal portion, a distal portion, and a cylindrical central passage therebetween; and the proximal portion including: a first recessed segment engaging the spring of the infusion device, a second recessed segment engaging the housing of the infusion device, and a third recessed segment engaging the cannula of the infusion device.
(B2) For the system denoted as (B1), the distal portion includes a central cavity defined by a valve flange, the central cavity operatively engaging the flow control valve.
(B3) For the system denoted as (B1) or (B2), the proximal portion further includes an axial wall for mechanically engaging the housing of the infusion device.
(B4) For the system denoted as any of (B1) through (B3), the valve flange includes a proximal face and a distal face defining a swage therebetween, the swage protruding inwardly into the central cavity.
(B5) For the system denoted as any of (B1) through (B4), the first recessed segment includes a plurality of septum protrusions extending proximally from a distalmost area of the first recessed segment.
(B6) For the system denoted as any of (B1) through (B5), the second recessed segment includes an angled portion and a flat portion for engaging the housing of the infusion device, the angled portion is radially angled outward at a proximal end; and the flat portion extends proximally from the angled portion.
(B7) For the system denoted as any of (B1) through (B6), the third recessed segment includes: a transverse groove and an angled wall proximal to the transverse groove, the angled wall tapering radially outward, a center of the transverse groove defines a larger diameter than a diameter of the third recessed segment, and the transverse groove and the angled wall mechanically engage the cannula of the infusion device.
(C1) A valve assembly configured to couple to an infusion device, the valve assembly including: a coupler, a flow control valve, and a luer lock fitting, the coupler including a proximal portion, a distal portion, and a cylindrical central passage therebetween; and the distal portion including: a central cavity receiving the flow control valve therein, a valve flange defining the central cavity, the valve flange engaging the luer lock fitting, and a swage extending radially inward from the valve flange to the luer lock fitting.
(C2) For the valve assembly denoted as (C1), the proximal portion includes a plurality of recessed segments configured to receive the infusion device.
(C3) For the valve assembly denoted as (C1) or (C2), the distal portion further includes an axial wall mechanically engaging the luer lock fitting.
(C4) For the valve assembly denoted as any of (C1) through (C3), the swage is welded to a proximal segment of the luer lock fitting.
(C5) For the valve assembly denoted as any of (C1) through (C4), the central cavity further includes a valve wall at a proximalmost area of the distal portion, the valve wall engages the flow control valve.
(C6) For the valve assembly denoted as any of (C1) through (C5), the valve assembly further includes a housing protrusion extending distally from the valve wall into the central cavity, the housing protrusion engages the flow control valve.

Although the invention has been described with reference to the embodiments illustrated in the attached drawing figures, it is noted that equivalents may be employed and substitutions made herein without departing from the scope of the invention as recited in the claims.

Having thus described various embodiments of the invention, what is claimed as new and desired to be protected by Letters Patent includes the following:

## Claims

1. A coupler for a valve assembly configured to couple to an infusion device, said coupler comprising:
a proximal portion, a distal portion, and a cylindrical central passage connecting the proximal portion and the distal portion,
said proximal portion comprising: a plurality of recessed segments configured to receive the infusion device,
wherein a first recessed segment of the plurality of recessed segments is configured to engage a spring of the infusion device,
wherein a second recessed segment of the plurality of recessed segments is configured to engage a housing of the infusion device,
wherein a third recessed segment of the plurality of recessed segments is configured to engage a cannula of the infusion device; and
said distal portion comprising:
a central cavity defined by a valve flange, wherein the central cavity is configured to engage a flow control valve.

2. The coupler of claim 1, wherein the proximal portion further comprises an axial wall configured to engage the housing of the infusion device.

3. The coupler of claim 1, wherein the valve flange comprises a proximal face and a distal face defining a swage therebetween, said swage protruding inwardly into the central cavity.

4. The coupler of claim 3, wherein the swage is configured to engage a luer lock fitting.

5. The coupler of claim 1, wherein the first recessed segment comprises a plurality of septum protrusions extending proximally from a distalmost area of the first recessed segment.

6. The coupler of claim 1, wherein the second recessed segment comprises an angled portion and a flat portion,
said angled portion being radially angled outward at a proximal end of the second recessed segment; and
said flat portion extending proximally from the angled portion.

7. The coupler of claim 1, wherein the third recessed segment comprises:
a transverse groove and an angled wall proximal to the transverse groove, said angled wall tapering radially outward,
wherein a center of the transverse groove defines a larger diameter than a diameter of the third recessed segment, and
wherein the transverse groove and the angled wall mechanically engage the cannula of the infusion device.

8. A system for controlling the flow of liquids to and from a patient, comprising:
a valve assembly and an infusion device,
said infusion device comprising a housing, a cannula, and a spring;
said valve assembly comprising a coupler and a flow control valve;
said coupler comprising a proximal portion, a distal portion, and a cylindrical central passage therebetween; and
said proximal portion comprising:
a first recessed segment engaging the spring of the infusion device,
a second recessed segment engaging the housing of the infusion device, and
a third recessed segment engaging the cannula of the infusion device.

9. The system of claim 8, wherein the distal portion comprises a central cavity defined by a valve flange, said central cavity operatively engaging the flow control valve.

10. A valve assembly configured to couple to an infusion device, said valve assembly comprising:
a coupler, a flow control valve, and a luer lock fitting,
said coupler comprising a proximal portion, a distal portion, and a cylindrical central passage therebetween; and
said distal portion comprising:
a central cavity receiving the flow control valve therein,
a valve flange defining the central cavity, said valve flange engaging the luer lock fitting, and
a swage extending radially inward from the valve flange to the luer lock fitting.

11. The valve assembly of claim 10, wherein the proximal portion comprises a plurality of recessed segments configured to receive the infusion device.

12. The valve assembly of claim 10, wherein the distal portion further comprises an axial wall mechanically engaging the luer lock fitting.

13. The valve assembly of claim 10, wherein the swage is welded to a proximal segment of the luer lock fitting.

14. The valve assembly of claim 10, wherein the central cavity further comprises a valve wall at a proximalmost area of the distal portion, wherein said valve wall engages the flow control valve.

15. The valve assembly of claim 14, further comprising a housing protrusion extending distally from the valve wall into the central cavity, wherein said housing protrusion engages the flow control valve.
